# EUROPÄISCHE PATENTSCHRIFT

(11) **EP 1 027 019 B1**
(45) Veröffentlichungstag und Bekanntmachung des Hinweises auf die Patenterteilung: **08.10.2003**
(21) Anmeldenummer: 98929323.8
(22) Anmeldetag: 15.05.1998
(51) Int. Cl.: A61F 2/46, B02C 19/12, B02C 19/20

(54) **VORRICHTUNG ZUM MAHLENDEN ZERKLEINERN VON GRANULATÄHNLICHEM HAUFWERK**
DEVICE FOR REDUCING GRANULATE-TYPE DEBRIS BY CRUSHING
DISPOSITIF POUR LE BROYAGE EN POUDRE DE DEBRIS DE GRANULATS

(30) Priorität: 31.10.1997 DE 29719385 U
(43) Veröffentlichungstag der Anmeldung: 16.08.2000
(73) Patentinhaber: Mondeal Medical Systems Gmbh, 78532 Tuttlingen (DE)
(72) Erfinder: DOMINGUEZ, Domingo, D-78532 Tuttlingen (DE); FRITZ, Arno, D-78570 Mühlheim (DE); SCHMID, Edwin, D-78594 Gunningen (DE)
(74) Vertreter: Hiebsch, Gerhard F., Dipl.-Ing.
(86) Internationale Anmeldenummer: EP9802874
(87) Internationale Veröffentlichungsnummer: WO99022676

(56) Entgegenhaltungen:
- WO-A-96/05914
- DE-A- 3 808 409
- DE-C- 3 734 714
- DE-C- 19 644 015
- DE-U- 29 719 385
- FR-A- 2 679 793
- FR-A- 2 712 483
- US-A- 4 706 897

## Beschreibung

Die Erfindung betrifft eine Vorrichtung zum mahlenden Zerkleinern von granulatähnlichem Haufwerk -- insbesondere von Knochenpartikeln, nach dem Oberbegriff des unabhängigen Patentanspruchs.

Mahlvorrichtungen der vorstehend genannten Art sind beispielsweise als Spongiosamühlen der DE 38 08 409 A1 zu entnehmen. Diese Spongiosamühle hat ein auf einen Vorratsschacht für das zu zerkleinernde Spongiosamaterial aufschiebbares Schiebergehäuse, dessen Innenfläche zusammen mit den Innenflächen des Vorratsschachtes eine bündig durchgehende Führung für einen Schieber bilden. Der Schieber wird durch einen Arbeitszylinder unter konstantem Druck gegen eine Zerkleinerungstrommel der Mühle zugestellt. Das Reaktionsteil dieses Arbeitszylinders ist mit dem Schiebergehäuse verbunden.

Mit der DE 37 34 714 wird ein Bioosteomikrotom vorgeschlagen, das mittels einer weitgehend rutschsicheren Grundplatte auf einem Arbeitstisch aufsitzt und dessen Schneidzylinder mittels eines verschwenkbaren Handhebelsbetätigt wird. Ein zwischen Handhebel und Schneidzylinder vorgesehenes Richtgesperre sorgt dafür, dass der Schneidzylinder nur bei Verschwenken des Handhebels in Richtung auf den Benutzer und die Grundplatte betätigt wird, während der Schneidzylinder bei entgegengesetzter Bewegung in seiner Position bleibt. Der Aufnahme des zerkleinerten Transplantates dient ein in den Schneidzylinder einschiebbarer Aufnahmebehälter.

In Kenntnis derartiger Mühlen hat sich der Erfinder das Ziel gesetzt, für den Einsatz im medizinischen Bereich eine verbesserte Mühle anzubieten, welche etwa aus granulatartigen Knochenpartikeln od.dgl. Haufwerk ein pulverartiges Mahlgut erzeugt.

Zur Lösung dieser Aufgabe führt die Lehre des unabhängigen Anspruches; die Unteransprüche geben günstige Weiterbildungen an.

Erfindungsgemäß ist der in einem -- an einem den Zuführschacht enthaltenden Sockelteil um eine Achse schwenkbar gelagerten -- bewegbaren Deckelteil des Gehäuses gleitbare Druckkörper an jenem von der Druckfläche fernen Ende von einer Feder als Kraftspeicher zur nachgebenden Druckvergleichmäßigung beaufschlagt. Zudem kann in weiterer Ausgestaltung der Druckkörper am Deckelteil in einem Winkel schwenkbar gelagert sein, so dass der in geöffnetem Zustand der Vorrichtung frei am klappbaren Deckelteil drehbare Druckkörper beim Schließvorgang dem Schachtmund zupendelt und in den Zuführschacht eingesenkt zu werden vermag. Die in Gebrauchsstellung dem Trommelmantel gegenüberstehende -entsprechend gekrümmte -- Druckfläche wird nach dem Einfüllen der Knochenpartikel od.dgl. in den Zuführschacht auf das Haufwerk aufgesetzt und übt auf dieses -- in Abhängigkeit von dessen Niveau -- gleichbleibenden Druck aus.

Vorteilhafterweise soll der Querschnitt eines jene Druckfläche anbietenden und im Zuführschacht geführten Querhauptes des Druckkörpers den Schachtquerschnitt mit Spiel ausfüllen, also die gesamte, den Schachtboden bildende teilzylindrische Fläche des Trommelmantes -- diesem in der Querschnittsform angepasst -- überlagern.

Der Zuführschacht soll mit dem seine Achse querenden -- die Mahltrommel aufnehmenden -- Kanal im quaderartigen Sockelteil des Gehäuses vorgesehen sein, das bevorzugt aus Aluminiumdruckguss gefertigt ist und dessen mit dem Mahlgut in Berührung kommende Innenflächen mit chirurgischem Stahl bekleidet sind, um toxische Verbindungen im Hinblick auf jene Leichtmetalllegierung des Gehäuses hintanzuhalten.

Der guten Bewegungsabläufe wegen soll das dem Kraftspeicher zugekehrte Ende des Druckkörpers in einem Lagerkörper vorgesehen werden, der mit Spiel schwenkbar in einer Einformung des Deckelteils angebracht ist. Bevorzugt besteht der Druckkörper aus dem genannten Querhaupt und einem an dieses -- unter Bildung schulterartiger Absätze -angeformten Gleitabschnitt, der in einem Führungsraum des Lagerkörpers eingreift und dort vorteilhafterweise mittels Kulissenelementen zwangsgeführt ist.

Der Lagerkörper kann um wenigstens eine quer zur Hubrichtung des Druckkörpers verlaufende Achse verschwenkt werden, die beidseits des Lagerkörpers in jeweils einem parallel zu Auflagekanten des Deckelteils in dessen Flankenwänden verlaufenden Langloch lagert, um die Bewegung des Druckkörpers der Lage des Schachtmundes anpassen zu können - falls gewünscht, kann zudem die Länge des Langloches durch wenigstens einen seinen Längsquerschnitt kürzenden Druckstift veränderbar sein.

Die Schwenkbewegung des Lagerkörpers bzw. des mit ihm verbundenen Druckkörpers kann durch Begrenzungsflächen der Einformung des Deckelteils begrenzt werden. Zudem soll in wenigstens einer Flankenwand -- ebenfalls zur Begrenzung der Schwenkbahn des Lagerkörpers -- ein Anschlagstift vorgesehen werden.

Im Rahmen der Erfindung liegt die besondere Ausgestaltung der Mahltrommel, die im Kanal des Mühlensockels mittels eines von einer ihrer Stirnseiten abragenden axialen Lagerstiftes in einer Lagerausnehmung des Sockelteils gelagert ist. Sie ist zudem andernends offen und ihre Mündung von einem Radialkragen umgeben, der in einen schulterartigen Ringabsatz des Sockelteils lagesichernd eingreift.

Außerhalb des Mühlensockels ist im Lagerstift eine Umfangsnut als Führungselement für einen die Dreheinrichtung bildenden Drehgriff vorgesehen; der Lagerstift polygonen Querschnittes ruht in einer entsprechenden Ausnehmung einer Griffhülse des Drehgriffes, wobei in jene Umfangsnut ein Halteelement des Drehgriffes radial eingreift; dieses Halteelement kann das freie Kugelende eines die Griffhülse teilweise durchsetzenden Klapphebels sein oder aber ein einfacher Radialstift.

Die Durchbrüche oder Bohrungen des Trommelmantels durchsetzen diesen des besseren Pulverdurchganges halber erfindungsgemäß in einem Neigungswinkel zu Durchmessergeraden der Mahltrommel, wobei dieser Neigungswinkel bevorzugt zwischen 40° und 50° -- insbesondere etwa bei 45° -- liegt. Die geometrischen Orte dieser Durchbrüche im siebartigen Trommelmantel werden im übrigen durch Knotenstellen eines Konstruktionsnetzes bestimmt, das von radialen Konstruktionslinien und diese in einem Winkel schneidenden weiteren Konstruktionslinien gebildet wird - diese messen bevorzugt etwa 50° bis 70°, insbesondere etwa 60°.

Insgesamt ergibt sich eine den Hygieneanforderungen zum einen sowie dem gewünschten Zerkleinerungsverhalten hohen Grades gerecht werdende Mühle.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1:: eine Schrägsicht auf eine erfindungsgemäße Mahlvorrichtung in geöffnetem Zustand mit seitlich abragendem Drehgriff;
- Fig. 2:: eine teilweise geschnittene Seitenansicht der Mahlvorrichtung in geschlossener Mahlstellung;
- Fig. 3:: eine gegenüber Fig. 1 vergrößerte und teilweise geschnittene Mahltrommel als Detail der Mahlvorrichtung;
- Fig. 4:: einen Teil der Mahltrommel in Draufsicht;
- Fig. 5:: einen Teilquerschnitt durch Fig. 4 nach deren Linie V - V;
- Fig. 6:: einen Teil der Fig. 2 in einer gegenüber dieser anderen Anordnung;
- Fig. 7:: einen vergrößerten Abschnitt eines Details der Fig. 6;
- Fig. 8:: eine weitere Ausgestaltung des Drehgriffes.

Eine Mahlvorrichtung oder Mühle 10 zum Zerkleinern von -- aus Gründen der Übersichtlichkeit in der Zeichnung nicht wiedergegebenen -- chirurgischen Knochenteilen weist auf einem gehäuseartigen Sockelteil oder Mühlensockel 12 etwa quaderförmiger Gestalt einen an einer Gelenkzunge 14 des Mühlensockels 12 durch wenigstens einen Achsstift 16 angelenkten Deckelteil oder Mühlendeckel 18 auf, das/der den Mühlensockel 12 zu einem Gehäuse ergänzt.

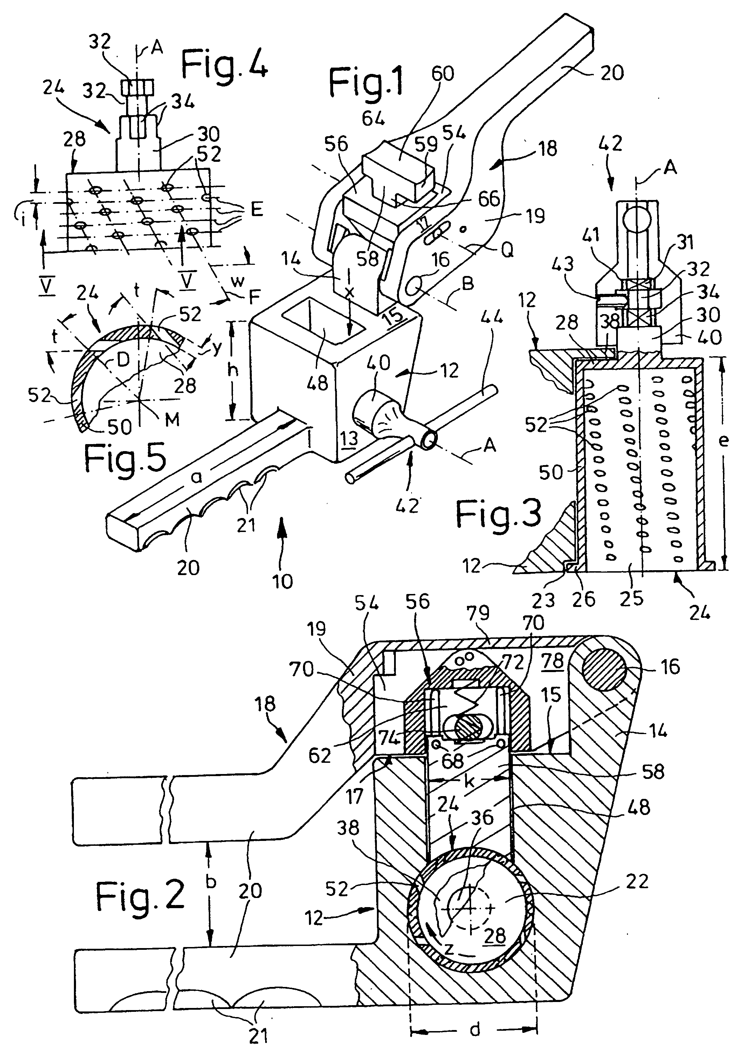

Sowohl vom Mühlensockel 12 -- der Höhe h von etwa 60 mm -- als auch vom Mühlendeckel 18 ragt ein querschnittlich etwa rechteckiges Griffprofil 20 der Länge a von etwa 120 mm ab; beide Griffprofile 20 verlaufen in der in Fig. 2 dargestellten Schließstellung der Mühle 10 in Abstand b von etwa 30 mm parallel zueinander. Das Griffprofil 20 des Mühlensockels 12 ist der besseren Handhabung wegen umlaufend mit Griffkehlen 21 ausgestattet.

Den Mühlensockel 12 durchsetzt -- quer zu den Griffprofilen 20 -- ein rohrartiger Kanal 22 des Durchmessers d von beispielsweise 30 mm, der eine Mahltrommel 24 aufnimmt. Letztere ist an einer Stirnseite offen und die so entstehende, die Trommelachse A querende Mündung 25 von einem ringartigen Radialkragen 26 umgeben. Dieser befindet sich in Einbaulage der Mahltrommel 24 in einem schulterartigen Ringabsatz 23, der -- den Kanal 22 umfangend -- in eine Seitenfläche 13 des Mahlsockels 12 eingeformt ist. Andernends ist die Mahltrommel 24 der axialen Länge e von etwa 60 mm mit einer Stirnplatte 28 verschlossen, von der ein axialer Lagerstift 30 abragt. In den Lagerstift 30 ist -- unter Bildung einer Endplatte 31 -- eine Umfangsnut 32 eingeformt; an diese schließen trommelwärts vier Abflachungen 34 des Lagerstiftes 30 an, die i. w. einen Vierkant erzeugen.

Beim Zusammenbau der Mühle 10 wird die Mahltrommel 24 in jenen Kanal 22 eingeführt und ihr Lagerstift 30 durch ein als Lagerausnehmung dienendes zentrisches Loch 36 einer den Kanal 22 -- als Teil der anderen Seitenfläche 13 -- einends abdeckenden Formplatte 38 hindurchgeschoben, bis die Stirnplatte 28 dieser Formplatte 38 innenseitig sowie der Radialkragen 26 dem beschriebenen schulterartigen Absatz 23 anliegen. Dann wird von außen her auf den Lagerstift 30 eine Griffhülse 40 eines Drehgriffes 42 aufgesetzt, bis jener vierkantartige Bereich des Lagerstiftes 30 in einer entsprechenden Ausnehmung 41 der Griffhülse 40 sitzt. Dann wird der Drehgriff 42 gemäß Fig. 3 mittels einer in die Umfangsnut 32 einragenden Radialschraube 43 an Lagerstift 30 so festgelegt, daß beim Drehen des Drehgriffes 42 über einen ihn diametral durchsetzenden Speichenstab 44 die Mahltrommel 24 mitgedreht wird.

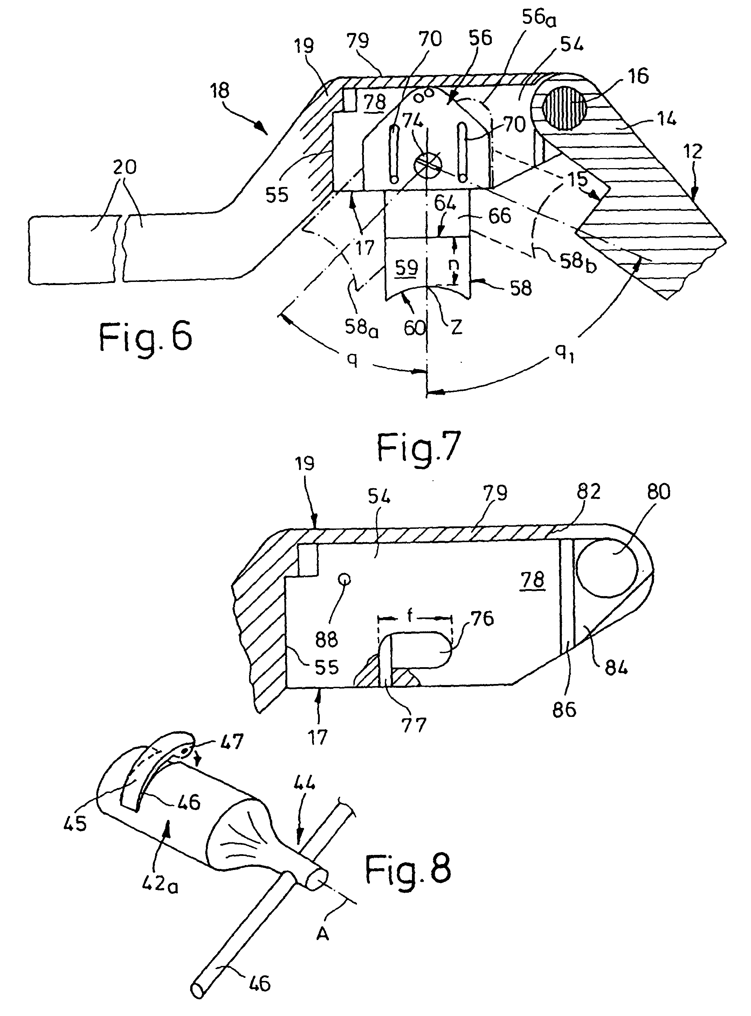

Beim Ausführungsbeispiel eines Drehgriffs 42ₐ der Fig. 8 ist statt jener Radialschraube 43 ein parallel zum Speichenstab 44 verlaufender Hebel 45 in einer schlitzartigen Ausnehmung 46 einends klappbar angelenkt, der andernends eine Rastkugel 47 trägt; diese sitzt in Gebrauchsstellung des Drehgriffs 42ₐ in der Umfangsnut 32 des Lagerstiftes 30.

Wie insbesondere Fig. 2 verdeutlicht, dreht sich die Mahltrommel 24 im Mühlensockel 12 an einem in diesen eingeformten -- querschnittlich rechteckigen -- Zuführschacht 48 der Breite k (k < d). Die Mahltrommel 24 bildet dabei einen in Drehrichtung z gekrümmten -- siebartigen -- Boden für den Zuführschacht 48; im zylindrischen Trommelmantel 50 der Mahltrommel 24 ist ein Muster von Durchbrüchen 52 vorgesehen.

Vor allem Fig. 4,5 ist das Muster dieser Durchbrüche 52 zu entnehmen; ein Netz von radialen Konstruktionslinien E, die von Radialebenen am Trommelmantel 50 bestimmt sind sowie in einem Abstand i von beispielsweise 4 mm verlaufen, werden von Konstruktionslinien F in einem Winkel w von etwa 60° gekreuzt. Dieses Netz aus Konstruktionslinien E,F bestimmt dann mit deren Kreuzungspunkten die geometrischen Orte der Durchbrüche 52. Diese durchsetzen als Bohrungen den Trommelmantel 50 der Dicke y von etwa 2 mm in einem querschnittlichen winkel t zu Durchmessergeraden D; dieser Winkel t mißt etwa 45°. Mit M ist in Fig. 5 der Querschnittsmittelpunkt des Trommelmantels 50 bezeichnet, der in jener Trommelachse A liegt.

Der Mühlendeckel 18 ruht in seiner Schließstellung mit seitlichen Auflagekanten 17 eines Deckelkopfes 19 auf Stirnflächen 15 des Mühlensockels 12 und setzt dessen Körper fort. Der Mühlendeckel 18 ist mit einer Einformung 54 versehen, welche in Schließstellung der Mühle 10 -- Fig. 2 -- oberhalb der Stirnfläche 15 des Mühlensockels 12 angeordnet ist. Diese Einformung 54 nimmt einen Lagerkörper 56 für einen in den Zuführschacht 48 absenkbaren und ihn querschnittlich ausfüllenden Druckkörper 58 auf; dessen der Mühlentrommel 24 zugeordnete teilzylindrisch gekrümmte Druckfläche 60 ist dem Außendurchmesser d des Trommelmantels 50 angepaßt.

In Abstand n von etwa 12 mm zum Tiefsten Z der Druckfläche 60 bietet der Druckkörper 58 beidends schulterartige Absätze 64 an; der Druckkörper 58 ist etwa "T"-förmig gestaltet mit einem von jener Druckfläche 60 begrenzten Querhaupt 59 und einem von den Absätzen 64 flankierten stempelartigen Gleitabschnitt 66, der in jenen Führungsraum 62 einragt. Im Führungsraum 62 des klotzartigen -- an der von der Mühlentrommel 24 weggekehrten Seite wie ein Satteldach geformten -- Lagerkörpers 56 ist der Gleitabschnitt 66 mit seitlichen Stiften 68 in schlitzartigen Gleitkulissen 70 gegen einen Kraftspeicher 72 geführt, beispielsweise gegen eine Schraubenfeder.

Parallel zur Achse B jenes den Mühlensockel 12 mit dem Mühlendeckel 18 verbindenen Achsstiftes 16 ragen aus dem Lagerkörper 56 Schraubbolzen 74 beidseits jeweils in ein Langloch 76 der die Einformung 54 begrenzenden Flankenwände 78 des Deckelkopfes 19 ein. Wie Fig. 7 verdeutlicht, ist jedes Langloch 76 parallel zu der die Flankenwände 78 verbindenden Dachplatte 79 des Deckelkopfes 19 angeordnet. Gemäß Fig. 7 kann bei einer Ausführung an einer Schmalseite des Langloches 76 dessen Länge f durch einen Drehstift 77 verkürzbar sein.

Die Dachplatte 79 endet in Abstand zum Achsloch 80 der Flankenwand 78 mit einer zur Einformung 54 geneigten Kante 82. Zwischen dieser und jenem Achsloch 80 setzt eine den Querschnitt der Einformung 54 verkürzende Anformung 84 der Flankenwand 78 an. Diese beidseitigen Anformungen 84 bilden - - parallel zum Drehstift 77 verlaufende -- Anschlagkanten 86 für den um die Achse Q der Schraubstifte 74 schwenkbaren Lagerkörper 56.

Dazu gibt Fig. 6 -- beidseits der hier vertikalen Lage in Schließstellung der Mühle 10 -- jeweils eine Endstellung des Druckkörpers 58 beim Verschwenken des Lagerkörpers 56 wieder. In Fig. 6 links ist dazu ein Schwenkwinkel q von etwa 45° für den strichpunktiert dargestellten Lagerkörper 56ₐ bzw. den Druckkörper 58ₐ zu erkennen, wenn dieser an eine Wandfläche 55 der Einformung 54 anschlägt. Die andere gestrichelt angedeutete Endlage 58_{b} des Druckkörpers 58 wird -- wie beschrieben -- von den Anschlagkanten 86 der Anformungen 84 mit einem Schwenkwinkel q₁ von hier etwa 70.° bestimmt.

Die Mühle 10 dient vor allem dem Erzeugen von Knochenmehl für chirurgische Eingriffe. Die Knochenpartikel werden bei geöffneter Mühle 10 gemäß Fig. 1 in Einführrichtung x in den Zuführschacht 48 auf den Trommelmantel 52 aufgebracht. Beim Schließen der Mühle 10 gelangt der pendelbare Druckkörper 58 in den Zuführschacht 48 und beginnt, die Knochenpartikel gegen den Trommelmantel 52 zu drücken. Dank der/des Kraftspeicher/s 72 wird der Andruck der Druckfläche 60 -- bei gleichbleibendem Druck durch den Anwender auf die Griffprofile 20 -- in Abhängigkeit von der Dicke der Partikelschicht geregelt; so kann der Anwender unabhängig von der Dicke der Partikelschicht die Mühle 10 stets mit gleichbleibender Kraft beaufschlagen.

In Fig. 7 ist noch einer von zwei Anschlag- oder Bremsstiften 88 zu erkennen, der die Flankenwand 78 durchgreift und eine Neigungsstellung des Lagerkörpers 56 fixieren hilft; die Anschlagstifte 88 sind zum Einstellen des genauen Schwenkradius des Druckkörpers 58 bzw. dessen Einführung in den Zuführschacht 48 notwendig.

Die Flächen des Zuführschachtes 48, des Druckkörpers 60 und des rohrartigen Kanals 22 sind mit rostfreiem chirurgischem Stahl ausgekleidet, um ein Kontaminieren des Knochenmaterials mit Abrieb des aus einer Aluminiumlegierung druckgegossenen Mühlensockels 12 hintanzuhalten. Diese Verkleidung kann eine Beschichtung oder ein in den Zuführschacht 48 bzw. den Kanal 22 eingesetztes und dort fixiertes Stahlrohr sein, das in der Zeichnung aus Gründen der Übersichtlichkeit nicht dargestellt ist.

## Patentansprüche

1. Vorrichtung zum mahlenden Zerkleinern von granulatähnlichem Haufwerk, insbesondere von Knochenpartikeln, mit in einem kanalartigen Raum (22) eines Gehäuses (12, 18) gelagerter Mahltrommel (24), die einen siebartig perforierten Trommelmantel (50) aufweist sowie durch eine außerhalb des Gehäuses (12, 18) angeordnete Dreheinrichtung (42) um ihre Trommelachse (A) bewegbar ist, wobei der Trommelmantel (50) den Boden eines im Gehäuse vorgesehenen Zuführschachtes (48) bildet und in letzterem ein mit einer in Gebrauchsstellung dem Trommelmantel (50) gegenüberstehenden Druckfläche (60) ausgestatteter sowie an seinem von der Druckfläche (60) fernen Ende von zumindest einem Kraftspeicher (72) beaufschlagbarer beweglicher Druckkörper (58) angeordnet ist, dessen quer zur Trommelachse (A) verlaufende Breite geringer ist als-der Durchmesser (d) des Trommelmantels (50),
**dadurch gekennzeichnet,**
**dass** der in einem an einem den Zuführschacht (48) enthaltenden Sockelteil (12) des Gehäuses (12, 18) um eine Achse (B) schwenkbar gelagerten Deckelteil (18) des Gehäuses (12, 18) gleitbare Druckkörper (58) an jenem von der Druckfläche (60) fernen Ende von einer Feder als Kraftspeicher (72) beaufschlagt ist.

2. Vorrichtung nach Anspruch 1, **dadurch gekennzeichnet, dass** der Druckkörper (58) am Deckelteil (18) schwenkbar (Winkel q, q₁) gelagert ist.

3. Vorrichtung mit querschnittlich entsprechend der Kontur des zylindrischen Trommelmantels (50) gekrümmter Druckfläche (60) nach Anspruch 1 oder 2, **dadurch gekennzeichnet, dass** der Querschnitt eines die. Druckfläche (60) enthaltenden und im Zuführschacht (48) geführten Querhauptes (59) des Druckkörpers (58) den Schachtquerschnitt mit Spiel ausfüllt.

4. Vorrichtung nach einem der Ansprüche 1 bis 3, **dadurch gekennzeichnet, dass** das dem Kraftspeicher (72) zugekehrte Ende des Druckkörpers (58) in einem Lagerkörper (56) vorgesehen und dieser mit Spiel schwenkbar in einer Einformung (54) des Deckelteils (18) angebracht ist.

5. Vorrichtung nach Anspruch 3 oder 4, **dadurch gekennzeichnet, dass** der Druckkörper (58) aus jenem Querhaupt (59) und einem an dieses unter Bildung schulterartiger Absätze (64) angeformten Gleitabschnitt (66) besteht, welcher in einen Führungsraum. (62) des Lagerkörpers (56) eingreift.

6. Vorrichtung nach Anspruch 4 oder 5, **dadurch gekennzeichnet, dass** der Lagerkörper (56) um eine quer zur Hubrichtung des Druckkörpers (58) verlaufende Achse (Q) schwenkbar ist und diese beidseits des Lagerkörpers (56) in jeweils einem parallel zu Auflagekanten (17) des Deckelteils (18) in dessen Flankenwänden (78) verlaufenden Langloch (76) lagert.

7. Vorrichtung nach Anspruch 6, **dadurch gekennzeichnet, dass** die Länge (f) des Langloches (76) durch wenigstens ein stiftartiges Element (77) veränderbar ist.

8. Vorrichtung nach einem der Ansprüche 4 bis 7, **dadurch gekennzeichnet, dass** Begrenzungsflächen (55, 86) der Einformung (54) des Deckelteils (18) den Schwenkbereich des Druckkörpers (58) bestimmen.

9. Vorrichtung nach einem der Ansprüche 4 bis 8, **dadurch gekennzeichnet, dass** in wenigstens einer Flankenwand (78) zur Begrenzung der schwenkbahn des Lagerkörpers (56) ein Anschlagstift (88) vorgesehen ist.

10. Vorrichtung nach einem der Ansprüche 4 bis 9, **dadurch gekennzeichnet, dass** das dem Kraftspeicher (72) zugekehrte Ende des Druckkörpers (58) im Führungsraum (62) des Lagerkörpers (56) durch Kulissenelemente (68, 70) gegen den Kraftspeicher (72) anhebbar geführt ist.

11. Vorrichtung mit von einer ihrer Stirnseiten abragendem axialem Lagerstift (30) im Gehäuse (12) gelagerter Mahltrommel (24) nach einem der Ansprüche 1 bis 10, **dadurch gekennzeichnet, dass** die andernends offene Mahltrommel (24) mit einem ihre Mündung (25) umgebenden Radialkragen (26) in einem schulterartigen Ringabsatz (23) des Sockelteils (12) gelagert ist.

12. Vorrichtung nach Anspruch 11, **dadurch gekennzeichnet, dass** der Lagerstift (30) außerhalb des Sockelteils (12) eine Umfangsnut (32) als Führungselement für einen Drehgriff (42) als Dreheinrichtung aufweist.

13. Vorrichtung nach Anspruch 11 oder 12, **dadurch gekennzeichnet, dass** der Lagerstift (30) polygonen Querschnittes undrehbar in einer entsprechenden Ausnehmung (41) einer Griffhülse (40) des Drehgriffes (42) ruht, wobei in die Umfangsnut (32) radial ein Halteelement (43, 47) des Drehgriffes (42) eingreift, das an der Griffhülse (40) des Drehgriffes (42) als das freie Ende eines die Griffhülse (40) teilweise durchsetzenden Klapphebels (45) ausgebildet ist.

14. Vorrichtung nach wenigstens einem der Ansprüche 1 bis 13, **dadurch gekennzeichnet, dass** die Lagen der Durchbrüche oder Bohrungen (52) im siebartigen Trommelmantel (50) durch Knotenstellen -eines Konstruktionsnetzes bestimmt sind, das durch von Radialebenen am Trommelmantel (50) erzeugten Konstruktionslinien (E) und diese im einem Winkel (w) schneidenden weiteren Konstruktionslinien (F) auf den Trommelmantel (50) gebildet ist, wobei dieser Winkel (w) bevorzugt etwa 50° bis 70°, insbesondere etwa 60°, mißt.

15. Vorrichtung nach Anspruch 14, **dadurch gekennzeichnet, dass** die Durchbrüche oder Bohrungen (52) den Trommelmantel (50) der Dicke (y) in einem Neigungwinkel (t) zu Durchmessergeraden (D) der Mahltrommel (24) durchsetzen, wobei der Neigungswinkel bevorzugt zwischen 40° und 50°, insbesondere etwa 45°, misst.

16. Vorrichtung nach wenigstens einem der Ansprüche 1 bis 15, **dadurch gekennzeichnet, dass** die vom Mahlgut berührbaren Flächen (22, 48, 60) aus rostfreiem Stahl bestehen oder mit diesem beschichtet sind.

17. Vorrichtung nach wenigstens einem der Ansprüche 1 bis 16, **dadurch gekennzeichnet, dass** sowohl vom Sockelteil (12) als auch vom Deckelteil (18) des Gehäuses (12, 18) jeweils ein Griffprofil (20) abragt und beide Griffprofile (20) in geschlossenem Zustand der Vorrichtung (10) ein parallel verlaufendes Griffpaar bilden.

## Claims

1. Device for pulverising by grinding of granulate-like debris, in particular of bone particles, with a grinding drum (24) held in a channel-like chamber (22) of a housing (12, 18) and having a drum casing (50), perforated like a sieve, and also being movable about its drum axis (A) by a turning device (42) arranged outside the housing (12, 18), wherein the drum casing (50) forms the floor of a feed shaft (48), provided in the housing, and arranged in the feed shaft is a movable pressure body (58), equipped with a pressure face (60) located opposite the drum casing (50) in the working position and able to be impacted by at least one energy store (72) at its end remote from the pressure face (60), the width of which pressure body running crosswise to the drum axis (A) is smaller than the diameter (d) of the drum casing (50), **characterised in that** the pressure body (58), slidable in a cover part (18) of the housing (12, 18) held as swivellable about an axis (B) on a base part (12) of the housing (12, 18) containing the feed shaft (48), is impacted by a spring as energy store (72) at the end remote from the pressure face (60).

2. Device according to claim 1, **characterised in that** the pressure body (58) is held as swivellable (angle q, q₁) on the cover part (18).

3. Device with a pressure face (60), curved in cross-section corresponding to the contour of the cylindrical drum casing (50), according to claim 1 or 2, **characterised in that** the cross-section of a crosshead (59) of the pressure body (58) containing the pressure face (60) and guided in the feed shaft (48) fills the cross-section of the shaft with play.

4. Device according to one of claims 1 to 3, **characterised in that** the end of the pressure body (58) facing the energy store (72) is provided in a bearing body (56) and this is mounted as swivellable with play in an indent (54) of the cover part (18).

5. Device according to claim 3 or 4, **characterised in that** the pressure body (58) consists of the afore-mentioned cross-head (59) and a sliding section (66) moulded on to it, forming shoulder-like steps (64), the sliding section engaging in a guide chamber (62) of the bearing body (56).

6. Device according to claim 4 or 5, **characterised in that** the bearing body (56) is swivellable about an axis (Q) running crosswise to the lifting direction of the pressure body (58), this axis being positioned on both sides of the bearing body (56) in each case in an elongated hole (76) running parallel to support edges (17) of the cover part (18) in its flank walls (78).

7. Device according to claim 6, **characterised in that** the length (f) of the elongated hole (76) can be altered by at least one pin-like element (77).

8. Device according to one of claims 4 to 7, **characterised in that** limiting faces (55, 86) of the indent (54) of the cover part (18) define the swivelling range of the pressure body (58).

9. Device according to one of claims 4 to 8, **characterised in that** in at least one flank wall (78) a stop pin (88) is provided for limiting the swivelling path of the bearing body (56).

10. Device according to one of claims 4 to 9, **characterised in that** the end of the pressure body (58) facing the energy store (72) is guided as capable of being lifted against the energy store (72) in the guide chamber (62) of the bearing body (56) by slotted link elements (68, 70).

11. Device with a grinding drum (24), held in the housing (12) by an axial bearing pin (30) projecting from one of its front sides, according to one of claims 1 to 10, **characterised in that** the grinding drum (24), open at the other end, is held by a radial collar (26) surrounding its outlet (25) in a shoulder-like annular step (23) of the base part (12).

12. Device according to claim 11, **characterised in that** outside the base part (12) the bearing pin (30) has a peripheral groove (32) as guide element for a turning handle (42) as turning device.

13. Device according to claim 11 or 12, **characterised in that** the bearing pin (30), of polygonal cross-section, rests as unrotatable in a corresponding recess (41) of the handle barrel (40) of the turning handle (42), wherein engaging radially in the peripheral groove (32) is a holding element (43, 47) of the turning handle (42), which is constructed on the handle barrel (40) of the turning handle (42) as the open end of a hinged lever (45) partially penetrating the handle barrel (40).

14. Device according to at least one of claims 1 to 13, **characterised in that** the positions of the openings or bores (52) in the sieve-like drum casing (50) are determined by nodal points of a structural network, formed on the drum casing (50) by construction lines (E) produced by radial planes on the drum casing (50) and further construction lines (F) bisecting them at an angle (w), this angle (w) measuring preferably approximately 50° to 70°, in particular approximately 60°.

15. Device according to claim 14, **characterised in that** the openings or bores (52) penetrate the drum casing (50) of thickness (y) at an angle of inclination (t) to diameter straight lines (D) of the grinding drum (24), the angle of inclination measuring preferably between 40° and 50°, in particular approximately 45°.

16. Device according to at least one of claims 1 to 15, **characterised in that** the faces (22, 48, 60) which can be touched by the grinding product consist of stainless steel or are coated therewith.

17. Device according to at least one of claims 1 to 16, **characterised in that** one handle section (20) projects in each case from both the base part (12) and from the cover part (18) of the housing (12, 18) and both handle sections (20) form a parallel running pair of handles in the closed state of the device (10).

## Revendications

1. Dispositif permettant de désintégrer par broyage des débris de type granulaire, en particulier des particules osseuses, avec un tambour de broyage (24) disposé dans une compartiment de type canal (22) d'une enceinte (12, 18), lequel tambour présente une enveloppe de tambour (50) perforée à la manière d'un tamis et peut se déplacer autour de son axe de tambour (A) par l'intermédiaire d'un dispositif rotatif (42) situé à l'extérieur de l'enceinte (12, 18), moyennant quoi l'enveloppe du tambour (50) forme le fond d'une cuve d'alimentation (48) prévue dans l'enceinte et dans cette dernière est disposée une coque pressurisée (58) mobile, équipé d'une surface de pression (60) opposée à l'enveloppe du tambour (50) dans la position d'utilisation et pouvant être sollicité par au moins un accumulateur d'énergie (72) à son extrémité éloignée de la surface de pression (60), la largeur du corps de pression transversale par rapport à l'axe du tambour (A) étant inférieure au diamètre (d) de l'enveloppe du tambour (50),
**caractérisé en ce que**, la coque pressurisée (58) pouvant coulisser dans un élément de couvercle (18) de l'enceinte (12, 18) disposé de manière pivotante autour d'un axe (B) au niveau d'un élément de socle (12) de l'enceinte (12, 18) contenant la cuve d'alimentation (48) peut être sollicitée par un ressort sous la forme d'un accumulateur d'énergie (72) à chaque extrémité éloignée de la surface de pression (60) .

2. Dispositif selon la revendication 1, **caractérisé en ce que** la coque pressurisée (58) est placé de manière à pivoter (angle q, q₁) sur l'élément de couvercle (18).

3. Dispositif avec une surface de pression (60) incurvée en coupe transversale suivant le contour de l'enveloppe du tambour cylindrique (50) selon la revendication 1 ou 2, **caractérisé en ce que** la section d'un chapeau de presse (59) de la coque pressurisée (58) contenant la surface de pression (60) et guidé dans la cuve d'alimentation (48) remplit la section de la cuve en laissant du jeu.

4. Dispositif selon l'une quelconque des revendications 1 à 3, **caractérisé en ce que** l'extrémité de la coque pressurisée (58) orientée vers l'accumulateur d'énergie (72) est prévue dans un coussinet de palier (56) et ce dernier est introduit de manière à pivoter dans un élément moulé (54) de l'élément de couvercle (18) en laissant du jeu.

5. Dispositif selon la revendication 3 ou 4, **caractérisé en ce que** la coque pressurisée (58) est constitué de chaque section (59) et d'un tronçon coulissant (66) formé sur celle-ci en formant des défauts de surface (64) de type épaulement, lequel tronçon coulissant s'engrène dans un compartiment de guidage (62) du coussinet de palier (56).

6. Dispositif selon la revendication 4 ou 5, **caractérisé en ce que** le coussinet de palier (56) peut osciller autour d'un axe (Q) transversal par rapport à la direction de levage de la coque pressurisée (58) et il est placé, des deux côtés du coussinet de palier (56), dans chaque fois un trou longitudinal (76) parallèle par rapport aux bords d'appui (17) de l'élément de couvercle (18) dans ses parois latérales (78) .

7. Dispositif selon la revendication 6, **caractérisé en ce que** la longueur (f) du trou longitudinal (76) peut être modifiée par au moins un élément de type tige (77).

8. Dispositif selon l'une quelconque des revendications 4 à 7, **caractérisé en ce que** les surfaces de délimitation (55, 86) de l'élément moulé (54) de l'élément de couvercle (18) déterminent la zone d'oscillation de la coque pressurisée (58) .

9. Dispositif selon l'une quelconque des revendications 4 à 8, **caractérisé en ce que** dans au moins une paroi latérale (78) permettant de délimiter la trajectoire d'oscillation du coussinet de palier (56), il est prévu une tige de butée (88).

10. Dispositif selon l'une quelconque des revendications 4 à 9, **caractérisé en ce que** l'extrémité orientée vers l'accumulateur d'énergie (72) de la coque pressurisée (58) est guidée et peut être soulevée dans le compartiment de guidage (62) du coussinet de palier (56) par l'intermédiaire d'éléments de coulisse (68, 70) contre l'accumulateur d'énergie (72).

11. Dispositif présentant un tambour de broyage (24) placé dans l'enceinte (12) par un tenon (30) axial dépassant de ses côtés frontaux selon l'une quelconque des revendications 1 à 10, **caractérisé en ce que** le tambour de broyage (24) ouvert au niveau de l'autre extrémité est placé dans un renfoncement annulaire de type épaulement (23) de l'élément de socle (12), une collerette radiale (26) entourant son embouchure (25).

12. Dispositif selon la revendication 11, **caractérisé en ce que** le tenon (10) présente en dehors de l'élément de socle (12) une rainure périphérique (32) jouant le rôle d'élément de guidage pour une poignée rotative (42) jouant le rôle de dispositif rotatif.

13. Dispositif selon la revendication 11 ou 12, **caractérisé en ce que** le tenon (30) présentant une coupe polygonale repose, sans pouvoir tourner, dans un évidement (41) correspondant d'un manchon de poignée (40) de la poignée rotative (42), moyennant quoi un élément de retenue (43, 47) de la poignée rotative (42) s'engrène radialement dans la rainure périphérique (42), lequel élément de retenue est formé sur le manchon (40) de la poignée rotative (42) en tant qu'extrémité libre d'un levier articulé (45) traversant en partie le manchon de la poignée (40).

14. Dispositif selon l'une quelconque des revendications 1 à 13, **caractérisé en ce que** les positions des perforations ou alésages (52) sont déterminées dans l'enveloppe du tambour de type tamis (50) grâce à aux emplacements des noeuds d'un réseau de construction, qui est formé par l'intermédiaire des lignes de construction (E) produites par les plans radiaux sur l'enveloppe du tambour (50) et d'autres lignes de construction (F) coupant celles-ci selon un angle (w) sur l'enveloppe du tambour (50), moyennant quoi cet angle (w) est de préférence compris entre 50° et 70°, et est en particulier égal à 60°.

15. Dispositif selon la revendication 14, **caractérisé en ce que** les perforations ou alésages (52) traversent l'enveloppe du tambour (50) de l'épaisseur (y) selon un angle d'inclinaison (t) par rapport aux droites du diamètre (D) du tambour de broyage (24), moyennant quoi l'angle d'inclinaison est de préférence compris entre 40° et 50°, et est en particulier égal à environ 45°.

16. Dispositif selon au moins l'une quelconque des revendications 1 à 15, **caractérisé en ce que** les surfaces (22, 48, 60) pouvant entrer en contact avec la substance à broyer sont réalisée en acier inoxydable ou en sont revêtues.

17. Dispositif selon au moins l'une quelconque des revendications 1 à 16, **caractérisé en ce que** chaque fois un profil de poignée dépasse aussi bien de l'élément de socle (12) que de l'élément de couvercle (18) de l'enceinte (12, 18) et les deux profils de poignée (20) forment une paire de poignées parallèles dans l'état fermé du dispositif (10).
